(19) Europäisches Patentamt
European Patent Office
Office européen des brevets

(11) Numéro de publication: **0 363 263 B1**

(12) **FASCICULE DE BREVET EUROPEEN**

(45) Date de publication de fascicule du brevet: **24.11.93**

(51) Int. Cl.5: **C07C 323/56**, C07C 319/14

(21) Numéro de dépôt: **89402701.0**

(22) Date de dépôt: **02.10.89**

(54) **Procédé de préparation de dérives de l'acide propionique.**

(30) Priorité: **03.10.88 FR 8812888**

(43) Date de publication de la demande:
**11.04.90 Bulletin 90/15**

(45) Mention de la délivrance du brevet:
**24.11.93 Bulletin 93/47**

(84) Etats contractants désignés:
**AT BE CH DE ES FR GB GR IT LI LU NL SE**

(56) Documents cités:
**EP-A- 0 127 882**
**EP-A- 0 158 339**
**EP-A- 0 182 273**

(73) Titulaire: **SYNTHELABO**
**22, avenue Galilée**
**F-92350 Le Plessis-Robinson(FR)**

(72) Inventeur: **Rossey, Guy**
**10, rue Paul Verlaine**
**F-78960 Voisins le Bretonneux(FR)**
Inventeur: **Ugolini, Antonio**
**8, rue de Seine**
**F-78230 Le Pecq(FR)**
Inventeur: **Chekroun, Isaac**
**25Bis, rue des Econdeaux**
**F-93800 Epinay(FR)**
Inventeur: **Vartanian, Abkar**
**65, Boulevard Michelet**
**Hardicourt**
**F-78250 Meulan(FR)**
Inventeur: **Wick, Alexander**
**10, Boulevard des Plants**
**F-78860 St Nom la Breteche(FR)**

(74) Mandataire: **Ludwig, Jacques et al**
**SYNTHELABO,**
**Service Brevets,**
**B.P. 72**
**F-92352 Le Plessis Robinson Cédex (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 363 263 B1

**Description**

La présente invention a pour objet un procédé de préparation de dérivés de l'acide propionique.

Le procédé de l'invention permet de préparer directement, sous la forme de diastéréoisomères (2S,3S) optiquement actifs, les composés de formule générale (I)

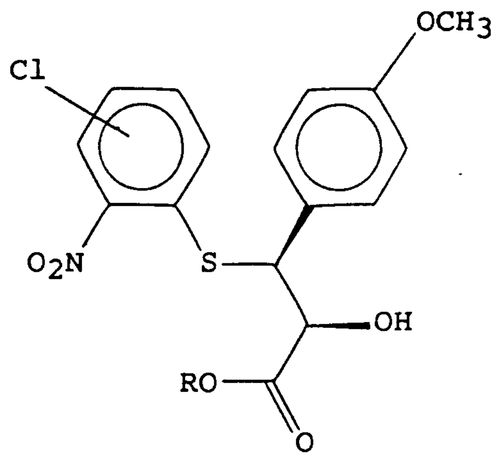

dans laquelle R est un atome d'hydrogène ou un radical $(C_{1-4})$alkyle.

Il consiste à faire réagir un thiophénol de formule générale (II)

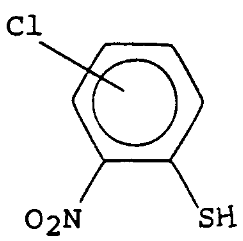

avec un composé de configuration 2R, 3S, de formule générale (III) dans laquelle R a la signfication ci-dessus

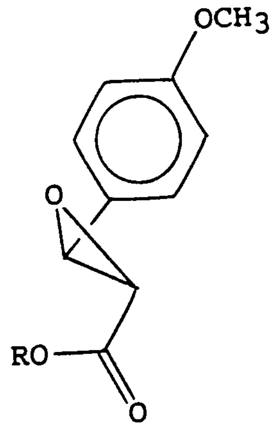

puis, si on le désire, on hydrolyse l'ester alkylique pour obtenir l'acide correspondant.

La réaction entre un composé (II) et un composé (III), qui sont tous deux décrits dans la littérature, peut être effectuée, à chaud, sous atmosphère inerte, dans un solvant inerte tel que le tétrahydrofuranne, le dioxanne, le nitrométhane, le diméthylformamide, un dichloroalcane, le toluène ou un solvant aromatique, pendant une durée de 1 à 10 h, selon la température de réaction.

Le principe et les conditions opérataires générales du procédé de l'invention sont décrits dans les demandes de brevets EP-A-0 127 882, EP-A-0 158 339 et EP-A-0 182 273.

Le composé de départ (III) (ester méthylique), sous la forme d'isomère (-) (2R,3S), est décrit dans les demandes de brevets japonais 145159/1986, 145160/1986 et 145174/1986.

Les composés (I) préparés selon le procédé de l'invention permettent de préparer des dérivés de benzothiazépinones, en particulier les dérivés chlorés du diltiazem.

Certains des composés (I) sont nouveaux et font partie de l'invention, en particulier l'acide (2S,3S) (chloro-6 nitro-2 phénylthio)-3 hydroxy-2 (méthoxy-4 phényl)-3 propionique.

Les exemples suivants illustrent l'invention. Les spectres IR et RMN et les analyses confirment la structure des composés.

Exemple 1.

(2S,3S) (Chloro-6 nitro-2 phénylthio)-3 hydroxy-2 (méthoxy-4 phényl)-3 propionate de méthyle.

Dans un ballon de 25 ml, muni d'un thermomètre et d'une arrivée d'azote, on place 22 mg d'acétate de zinc sous forme de dihydrate, 948 mg de chloro-6 nitro-2 thiophénol et 5 ml de toluène. A cette solution sous agitation, on ajoute goutte à goutte, en 5 mn, une solution de 1,093 g de (-) (2R,3S) (méthoxy-4 phényl)-3 époxy-2,3 propionate

de méthyle dans 7,5 ml de toluène à 25 °C.
La température monte à 27-28 °C. On maintient l'agitation pendant 1 h 30. Le mélange réactionnel est amené à sec sous vide. On obtient une huile jaune que l'on utilise telle quelle pour l'étape suivante décrite dans l'exemple 2.

Exemple 2.

Acide (+) (2S,3S) (chloro-6 nitro-2 phénylthio)-3 hydroxy-2 (méthoxy-4 phényl)-3 propionique

Dans un ballon de 50 ml, on place 2 g de l'ester obtenu dans l'exemple 1 que l'on dissout dans 10 ml de méthanol. A cette solution on ajoute 13 ml d'eau et 0,6 ml de soude 10N.
La solution est agitée à 25-28 °C pendant 3 h. Le mélange est refroidi au bain de glace puis acidifié avec HC1 concentré jusqu'à pH 1. On élimine le méthanol. Un produit gommeux précipite.
On laisse reposer au régrigérateur pendant 16 h. On filtre, lave à l'eau. L'insoluble gommeux est trituré avec quelques ml d'acétate d'éthyle, on obtient une suspension que l'on filtre, lave avec de l'acétate d'éthyle. Le produit est séché sous vide, on recueille l'acide brut, que l'on fait recristalliser dans 10 ml d'acétate d'éthyle à la température du reflux.
On obtient l'acide avec un rendement de 65,6 %.
F = 186-188 °C (cristaux jaunes)
$[\alpha]_D^{20}$ = + 76,3° (c = 0,5, méthanol)
$[\alpha]_D^{20}$ = + 27,6° (c = 0,5, NaOH à 1N).

Exemple 3.

(2S,3S) (Chloro-5 nitro-2 phenylthio)-3 hydroxy-2 (méthoxy-4 phenyl)-3 propionate de méthyle.

Dans un ballon de 25 ml muni d'un thermomètre, d'une arrivée d'azote, on introduit 26 mg d'acétate de zinc sous forme de dihydrate, 948 mg de chloro-5 nitro-2 thiophénol et 5 ml de toluène. A cette suspension, on ajoute, sous agitation et sous azote, en 5 mn, une solution de 1,093 g de (-) (2R,3S) (méthoxy-4 phényl)-3 époxy-2,3 propionate de méthyle dans 9 ml de toluène.
La température s'élève à 29 °C. On laisse agiter ainsi pendant 45 mn. Le mélange réactionnel est amené à sec sous vide. On obtient une huile jaune que l'on utilise telle quelle dans l'étape suivante décrite dans l'exemple 4.

Exemple 4.

Acide (+) (2S,3S) (chloro-5 nitro-2 phénylthio)-3 hydroxy-2 (méthoxy-4 phényl)-3 propionique.

Dans un ballon de 50 ml, on introduit 2 g de l'ester obtenu dans l'exemple 3 et 14 ml de méthanol puis 22 ml d'eau. Un produit précipite. Sous agitation, on ajoute 0,6 ml de soude 10N et agite pendant 3 h. On filtre le mélange réactionnel refroidit le filtrat en bain de glace, puis sous agitation on acidifie à pH 1 avec 0,7 ml d'acide chlorhydrique concentré. On filtre l'acide, le lave à l'eau puis le sèche sous vide en présence d'anhydride phosphorique pendant 16 h et à 50 °C pendant 4 h. On recueille 1,78 g d'acide brut (97,2 %) que l'on fait recristalliser dans 8 ml d'alcool isopropylique à la température du reflux. Le produit cristallise avec 0,2 mole d'alcool isopropylique.
F = 120-127 °C. (S'il reste 1 mole d'alcool isopropylique,
F = 92-97 °C, comme décrit dans la littérature).
$[\alpha]_D^{20}$ = + 132,5° (c = 0,5, chloroforme).

Les composés (I), préparés selon le procédé de l'invention, sont des intermédiaires de synthèse pour la préparation de benzothiazépinones chlorées, en particulier pour la préparation des dérivés chlorés du diltiazem, selon les modes opératoires décrits dans la littérature, par exemple dans le brevet français BSM 8199M, et par exemple dans la demande de brevet européen n° 0127882.

**Revendications**

1. Procédé de préparation de dérivés de l'acide propionique de configuration 2*S*,3*S* répondant à la formule générale (I)

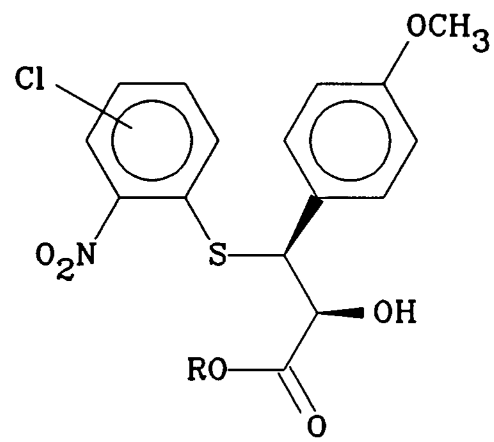

dans laquelle R est un atome d'hydrogène ou un radical ($C_{1-4}$)alkyle,
procédé caractérisé en ce que l'on fait réagir un composé de formule générale (II)

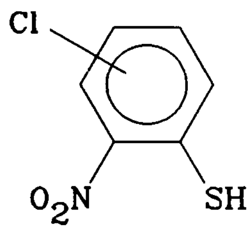

avec un composé de configuration *2R,3S* de formule générale (III)

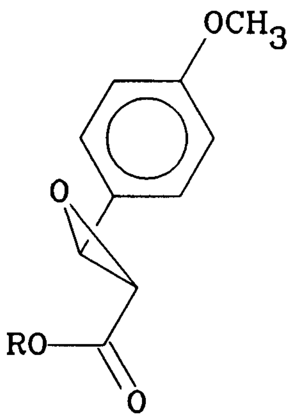

dans laquelle R a la signification ci-dessus, à chaud, sous atmosphère inerte, dans un solvant inerte tel que le tétrahydrofurane, le dioxanne, le nitrométhane, le diméthylformamide, un dichloroalcane, le toluène, un solvant aromatique, pendant une durée de 1 à 10 h, selon la température de réaction et, si on le désire, on hydrolyse l'ester alkylique obtenu en acide correspondant.

2. Procédé selon la revendication 1, caractérisé par le fait que R est un radical méthyle.

3. Procédé selon la revendication 1, caractérisé par le fait que le solvant est le toluène.

4. Procédé selon la revendication 2, caractérisé par le fait que la température de réaction entre les composés de formules générales (II) et (III) est de 20 à 30 °C.

5. L'acide (+) (2S,3S) (chloro-6 nitro-2 phénylthio)-3 hydroxy-2 (méthoxy-4 phényl)-3 propionique.

## Claims

1. Process for the preparation of propionic acid derivatives of 2S,3S configuration corresponding to the general formula (I)

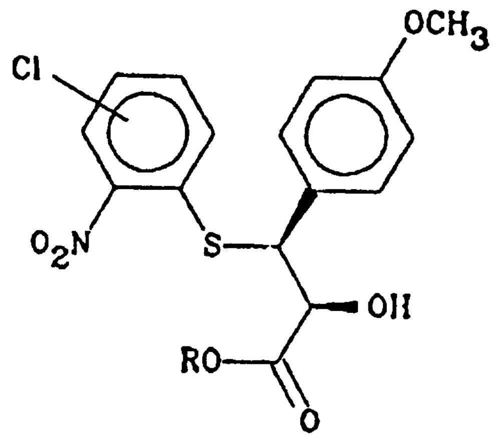

in which R is a hydrogen atom or a $(C_{1-4})$alkyl radical, which process is characterized in that a compound of general formula (II)

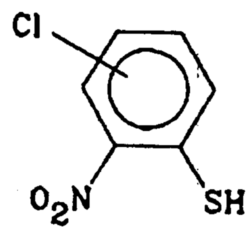

is reacted with a compound of 2R,3S configuration of general formula (III)

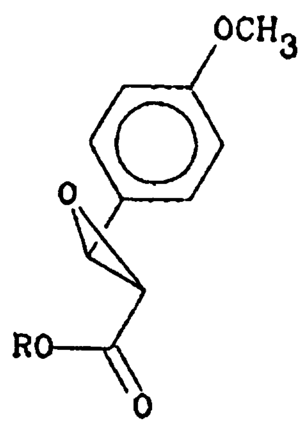

in which R has the meaning above, while hot, under an inert atmosphere, in an inert solvent such as tetrahydrofuran, dioxane, nitromethane, dimethylformamide, a dichloroalkane, toluene or an aromatic solvent, for a period of 1 to 10 h, depending on the reaction temperature, and, if desired, the alkyl ester obtained is hydrolyzed to the corresponding acid.

**2.** Process according to Claim 1, characterized in that R is a methyl radical.

**3.** Process according to Claim 1, characterized in that the solvent is toluene.

**4.** Process according to Claim 2, characterized in that the temperature of reaction between the compounds of general formulae (II) and (III) is from 20 to 30 ° C.

**5.** ( + )-(2S,3S)-3-(6-Chloro-2-nitrophenylthio)-2-hydroxy-3-(4-methoxphenyl)propionic acid.

**Patentansprüche**

**1.** Verfahren zur Herstellung von Propionsäurederivaten in der 2S,3S-Konfiguration der allgemeinen Formel (I)

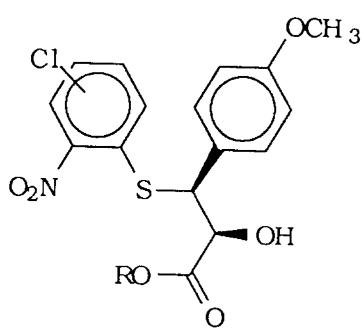

in der R ein Wasserstoffatom oder eine $C_{1-4}$-Alkylgruppe bedeutet, **dadurch gekennzeichnet**, daß man eine Verbindung der allgemeinen Formel (II)

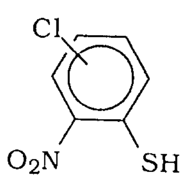

mit einer Verbindung der Konfiguration 2R,3S der allgemeinen Formel (III)

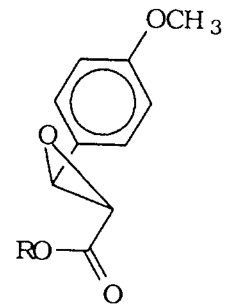

in der R die oben angegebenen Bedeutungen besitzt, in der Wärme unter einer inerten Atmosphäre in einem inerten Lösungsmittel, wie Tetrahydrofuran, Dioxan, Nitromethan, Dimethylformamid, einem Dichloralkan, Toluol oder einem aromatischen Lösungsmittel während einer Zeitdauer von 1 bis 10 Stunden in Abhängigkeit von der Reaktionstemperatur umsetzt und gewünschtenfalls den erhaltenen Alkylester zu der entsprechenden Säure hydrolysiert.

**2.** Verfahren nach Anspruch 1, **dadurch gekennzeichnet**, daß R eine Methylgruppe bedeutet.

**3.** Verfahren nach Anspruch 1, **dadurch gekennzeichnet**, daß das Lösungsmittel Toluol ist.

**4.** Verfahren nach Anspruch 2, **dadurch gekennzeichnet**, daß die Temperatur der Reaktion der Verbindungen der Formeln (II) und (III) 20 bis 30 ° C beträgt.

**5.** ( + )-(2S,3S)-3-(6-Chlor-2-nitrophenylthio)-2-hydroxy-3-(4-methoxyphenyl)-propionsäure.